# EUROPEAN PATENT SPECIFICATION

(11) **EP 2 373 219 B1**
(45) Date of publication and mention of the grant of the patent: **08.02.2017**
(21) Application number: 09764814.1
(22) Date of filing: 03.12.2009
(51) Int. Cl.: A61B 5/00, G06Q 99/00, A45D 44/00, A61B 5/103

(54) **EVALUATION AND SELECTION PROCESS FOR CONSUMER PRODUCTS**
BEWERTUNGS- UND AUSWAHLVERFAHREN FÜR KONSUMGÜTER
PROCÉDÉ D'ÉVALUATION ET DE SÉLECTION POUR PRODUITS DE CONSOMMATION

(30) Priority: 08.12.2008 US 330231
(43) Date of publication of application: 12.10.2011
(73) Proprietor: Unilever PLC, London EC4Y 0DY (GB); Unilever N.V., 3013 AL Rotterdam (NL)
(72) Inventor: VELTHUIZEN, Robert, Paul, Trumbull, CT 06611 (US); JIANG, Zhi-Xing, Trumbull, CT 06611 (US); DESSIRIER, Jean-Marc, Trumbull, CT 06611 (US); COVELL, Edwin, Ralph, Trumbull, CT 06611 (US)
(74) Representative: Corsten, Michael Allan
(86) International application number: PCT/EP2009/066322
(87) International publication number: WO 2010/066633

(56) References cited:
- EP-A- 1 248 237
- WO-A-2008/027533
- US-A1- 2007 255 589
- US-A1- 2008 214 907
- US-A1- 2008 273 745
- ZARA AMBADAR: "THE EFFECTS OF MOTION AND ORIENTATION ON PERCEPTION OF FACIAL EXPRESSIONS AND FACE RECOGNITION" INTERNET CITATION, [Online] 1 January 2002 (2002-01-01), page Complete, XP007911707 Retrieved from the Internet: URL:http://etd.library.pitt.edu/ETD/availa ble/etd-08202002-162757/unrestr icted/ambadar.pdf> [retrieved on 2010-02-12]
- FINK B ET AL: "Human (Homo Sapiens) Facial Attractivenesss in Relation to Skin Texture and Color" JOURNAL OF COMPARATIVE PSYCHOLOGY, AMERICAN PSYCHOLOGICAL ASSOCIATION, WASHINGTON, DC, US LNKD- DOI:10.1037//0735-7036.115.1.92, vol. 115, no. 1, 1 March 2001 (2001-03-01) , pages 92-99, XP007913398 ISSN: 0735-7036
- "Superb Skin Airbrush Technique / Photoshop Tutorials" INTERNET CITATION February 2008 (2008-02), pages 1-16, XP007913399 [retrieved on 2010-06-07]

## Description

### FIELD OF THE INVENTION

The present invention is directed to an evaluation and selection process for consumer products. More particularly, the invention is directed to an evaluation process that includes demonstrating the effectiveness of consumer product on a consumer's skin by comparing actual images of skin treated with product to images that were, for example, digitally transformed. The invention also includes the assessment of product differences and the assignment of a symbol associated with the differences in order to assist consumers with the selection of a 'product.

### BACKGROUND OF THE INVENTION

Many consumers, especially baby boomers, are concerned with their looks. Particularly, consumers are concerned with the characteristics of their skin on and around the face. Skin characteristics, including lightening, darkening, wrinkling, dryness and uneven hyperpigmentation are just some of the characteristics that concern consumers.

Many commercially available skin products are available to consumers and such commercially available products often take months to show a noticeable difference on skin after use. As a result of this, there is a need for a fast and convenient system that assists consumers in comparing products and selecting products that would be beneficial to them.

The present invention, therefore, is directed to an evaluation and selection process for consumer products. The invention allows for the comparison of products based on information generated by comparing actual images of skin treated with product to images that were, for example, digitally transformed. The invention also includes the assessment of product differences and the assignment of a symbol associated with the differences in order to assist consumers with the selection of product.

### ADDITIONAL INFORMATION

Efforts have been disclosed for evaluating consumer products. In US 6 959 119, described is a method for displaying images of a consumer's skin subjected to digital transforming.

Other efforts have been disclosed for assessing skin color attributes. In US 2004/0261280 A1, a color ruler device, method and kit are described. In US 2007/0086651 A1, a method and apparatus for characterizing imperfections of skin are described, and in WO 2008/028893 A1, a method for measuring blemishing on skin is described.

Still other efforts have been disclosed for assessing products. In US 6 734 858, a method for aging an image via an electronic interface to demonstrate a benefit is described.

Even other efforts have been disclosed for assessing skin. In JP 2007-252891 and JP 2002-330943 (abstracts), methods to evaluate skin for age and beauty, respectively, are described.

None of the additional information above describes an evaluation and selection process for consumer products where the process allows for the comparison of product based on information generated by comparing actual images of skin treated with product to images that were altered, for example, digitally. Moreover, none of the information above describes an assessment of product differences associated with a symbol for the purpose of assisting consumers with the selection of a product.

### SUMMARY OFTHE INVENTION

In a first aspect, the present invention is directed to a method for evaluating consumer products comprising the steps of:
(a) analyzing untreated skin of a consumer and artificially transforming the untreated skin to a desired result to produce artificially transformed skin by applying a cover-up or by using image transformation;
(b) treating the untreated skin with a product to produce treated skin; and
(c) comparing the treated skin to the artificially transformed skin to yield a difference associated with a symbol,
wherein at least two consumer products are evaluated and compared to artificially transformed skin, wherein the method is not a method for performing a mental act, or for treatment of the human body by surgery ortherapy.

In a preferred aspect, the present invention is directed to assessing skin evenness with blue light transform.

Analyzing, as used herein, means assessing, for example, by visually studying a portion of skin with or without a visual aid such as a magnifying glass. Analyzing also includes the digital analysis of skin by using, for example, photography and image analysis. Such analyzing is meant to be directed at skin traits or characteristics which include evenness of color, beauty, radiance, glow, texture, naturalness, pore size as well as moisture content. Skin, as used herein, is meant to include skin on the face, neck, chest, back, arms, hands, legs, scalp, feet, buttocks and abdomen. Artificially transformed means modified by a process that does not include using an active (i.e. a chemical compound) to produce an intended result. Artificially transformed, therefore, does include applying a cover-up such as a make-up to create a desired end result, or using image transformation that includes, for example, light, lenses, mirrors and/or digital transformation (using image arithmetic) to create a desired look. The transformation may be made to the skin itself, to a photo image of the skin and/or both. Symbol, as used herein, means an identifier that includes a number, shape, emblem or the like that may be used to identify a difference, and particularly, the perceived difference between the performance of one product against another product in comparison to artificially transformed skin. Such a symbol may be used in advertisements (written and/or oral) as well as on a product. Blue light transform, as used herein, means the utilization of blue light (i.e. light having or wavelength from about 440 to about 490 nm) to transform an image of skin, including a photograph thereof, by (arithmetically) adding red light (i.e. light having a wavelength from about 630 to about 670 nm) thereto. Evenness, as used herein, means the spatial homogeneity of skin color. Images of skin means an image created by observing skin or a reproduction thereof such as a photographic reproduction. Treated with a product includes product topically applied, ingested, injected, product applied via radiation with visible light or ultraviolet radiation or both, as well as product applied or resulting from treatment with a physical device such as those that employ microneedles or ultrasound. Product, as used herein, includes products to leave on skin as well as products to wash off. Comprising, as used herein, is meant to include consisting essentially and consisting of. Unless specifically stated otherwise, all ranges defined are meant to include all ranges subsumed therein.

### DETAILED DESCRIPTION OF THE PREFERRED EMBODIMENTS

The only limitation with respect to the type of skin characteristic that may be assessed with this invention is that the characteristic is of the type typically of concern for humans. When practicing the present invention, untreated skin, typically on the human body and preferably on the face, is assessed and analyzed for a targeted characteristic, like evenness of color, before being treated with a product or regimen designed to improve color evenness. The assessment may be of the skin directly or a photograph thereof. Panelist involved in the assessment may be asked a series of questions (i.e., surveyed) generally related to the targeted skin characteristic, where such questions may, for example, relate to the panelist's height, weight, gender, sex, age, diet, nationality, skin condition and/or desired skin improvements.

The information collected from the assessment, including any answer or answers provided during the survey, may, for example, result in a recommended product for the panelist to utilize in order to improve the targeted characteristic, like evenness of color. Subsequent to utilizing the product recommended, the resulting treated skin is assessed for product efficacy and overall performance. Any and all information generated pre- and post-treatment may be stored and maintained, preferably, in a database and can be used in association with a symbol to market products.

Typically, when product is used to yield treated skin, the product is used once, or for a period from about one (1) minute to about twelve (12) months, and most preferably, for a period from about two (2) minutes to about four (4) months, including all ranges subsumed therein. In addition to the above, the untreated skin or any photo or image thereof can be artificially transformed independent of using product to yield a desired consumer result as it relates to the targeted characteristic. In a preferred embodiment, the results from the artificial transformation are then compared to the results from the assessment of the treated skin. In a most preferred embodiment, skin targeted for analysis is artificially transformed independent of being treated so that the treated skin and artificially transformed skin originate from the same position/body part of the panelist. The assessment may be conducted by the panelist and thereby be a self assessment study or be conducted by individuals that can be observers that may or may not have been subjected to product or trained graders that are not panelists having their skin subjected to product and artificial transformation but are individuals with training and experience in assessing skin.

When assessing product, typically at least about ten (10) panelists, and preferably, from about 15 to about 150 panelists, and most preferably, from about 50 to about 100 panelists participate in the evaluation of a single product. When, for example, skin lightening products from competitors are being assessed, it is preferred that the assessments made or generated are the result of different products being applied to the same panelist. A first product, therefore, may be applied to, for instance, the right cheek of a panelist and a second product may be applied to the left cheek of the same panelist. Product A would thus be compared to product B by applying product to corresponding areas of skin of at least about ten (10) panelists. This allows for very accurate bilateral comparisons. In a preferred embodiment, at least two products targeted for an identical characteristic are compared, and most preferably, from about two (2) to about ten (10) products targeted for an identical characteristic are compared. In yet another preferred embodiment comparisons are made between skin at multiple time points (e.g. between 0-30 weeks, preferably 0-20 weeks) during treatment with the artificially transformed skin at multiple transform levels (e.g. 7-12% or 13-18%) by arithmetic blending according to the formula: Q(i,j) = X * P₁ (i,j) + (1-X) * P₂ (i,j) where i,j defines a pixel, P₁ and P₂ are two input images although P₂ can also be a constant, and X is a blending ratio or transform level determining the influence of each input image to the output image Q.

The product employed in this invention is limited only to the extent that it may be used with humans. Such a product can comprise, for example, skin lightening agents like niacinamide, and/or hydroquinone, resorcinols and its derivatives, retinoids, conjugated linoleic acid, petroselinic acid, alpha-hydroxy acids, beta-hydroxy acids, peroxides, fragrances, plant extracts, surfactants, humectants, thickeners, water, silicones, solvents, sunscreens, vitamins other than niacinamide, tea or tea solids, anti-acne agents, preservatives and any art recognized combination thereof.

It is within the scope of this invention to assign a symbol associated with the efficacy or performance of each product tested, and it is preferred that the untreated skin is artificially transformed to a desired result of the consumer (i.e. panelist) and compared to the actual results obtained by using product. For illustrative purposes and clarity, a consumer/panelist may select a particular facial trait, like evenness of color around the cheeks that he or she would like to improve. Artificial transformation may be employed on one or both of the cheeks of the consumer to yield a desired result. Subsequently, products of one company should be employed on one cheek of the panelist and the product of a second company on the opposite cheek of the same panelist. The results obtained may then be compared to the artificially transformed skin selected to mimic a desired result identified by the panelist. Subsequent to all panelists completing the regimen and the comparison of treated skin to the desired artificially transformed skin, the results obtained may be statistically analyzed to result in a perceived difference (delta) that may be used in advertising and to sell product.

In a preferred embodiment, the results obtained are generated by asking the panelists, observers or graders pointed questions in a two-alternative forced choice task. Such panelists or graders may be asked, for example, which image has more even color or which image has less wrinkles and pores? In another preferred embodiment, the results are generated by using conventional image analysis methods (e.g., like those made commercially available by Media Cybernetics and ITT Visual Information Solutions) on both transformed images from artificially transformed skin and images of treated skin.

The data analysis which may be employed in this invention is limited only to the extent that it is generally recognized when assessing consumer products and provides a numerical estimate of the perceived difference between the treated and transformed skin. Illustrative examples include D-prime, R-Index, Cohen's D and T-statistic.

As to the artificial transformation of untreated skin, the same may be achieved by digitally transforming an image as described in US 6 959 119 B2, as well as by using averaging techniques as described in Prototyping and Transforming Facial Textures for Perception Research, IEEE computer Graphics and Applications, Vol. 21, pp. 42-50, 2001, the disclosures of which are incorporated herein by reference. In a preferred embodiment, the characteristic targeted in this invention is evenness of skin color. In another preferred embodiment, the artificial transformation of such unevenly colored skin is achieved by using blue light and introducing through image arithmetic red light into the blue light channel to create the desired artificially transformed image. Typically, this method, identified herein as blue light transform, will require from about 0.5 to about 60%, and preferably, from about 3 to about 40%, and most preferably, from about 6 to about 30% red light in the blue light channel, including all ranges subsumed therein.

The output or perceived difference obtained in this invention and resulting from the assessment defined herein may be associated with a symbol in order to convey to a consumer the perceived difference between how a particular product performs against another product. To illustrate, if product A and product B are to be assessed for their ability to improve skin texture, every panelist involved would have her untreated skin analyzed and artificially transformed to a look suitable to her liking, where the artificial transformation is equally applied to each panelist.

Subsequent to being treated with product A and B (i.e., an identical regimen for each panelist), the perceived difference in skin treated with product and the artificially transformed skin results in a symbol. A one (1) or star, for example, can mean the product resulted in skin very close to the look of the artificially (highly desirable) transformed skin. A five (5) or a square can mean the product resulted in skin which was about fifty (50) percent improved when compared to artificially transformed skin. A zero (0) or a circle can mean no improvement when comparing treated skin to untreated skin and/or the artificially transformed skin.

The symbols used and the products associated with such symbols may be described in any art recognized advertisement, such as a magazine advertisement or in a television or radio commercial. Such symbols may be marked on the product of the company utilizing this invention whereby the consumer can connect with the symbol if he or she is familiar with the advertisement. Alternatively, a key or advertisement may be placed near product at a store so that a consumer can connect with the symbol prior to making a purchase.

The examples are provided to facilitate an understanding of the invention. The examples are not intended to limit the scope of the claims.

### Example 1

Forty-nine (49) panelists were subjected to blue light transform in order to yield facial images having a desired evenness of color (i.e. artificially transformed skin). The transform was achieved by blending 10% and 18% red channel into blue channel. Product, comprising hydroquinone (4%), was prescribed by a dermatologist to the forty-nine (49) panelists. The product was home-used once a day for twelve weeks and applied to the face of the panelists. After completion of product application, the panelists evaluated their photographs in a forced-choice experiment. At zero (0) weeks (i.e. before initial application of product) the 10% and 18% transforms had D-prime values of about 1.4 and 2.3, respectively, when compared to untreated skin. After five (5) weeks of treatment, the 10% and 18% transforms had D-prime values of about 0.6 and 1, respectively, when compared to the treated skin. After eight (8) weeks of treatment, the D-prime values for the 10% and 18% transforms were 0.1 and 0.6, respectively. At twelve (12) weeks, essentially no difference could be seen between the treated skin and the artificially transformed skin (10% and 18%). The D-prime values obtained herein may be used to create a system with symbols to assist in the marketing and advertising of consumer products, such as skin lightening products. After fitting a bi-linear model with transform level and time point as independent variables, it was seen that at twelve (12) weeks the product tested achieved the effect of a 13% blue light transform.

### Example 2

The procedure in example 2 was performed in a manner similar to the one described in example 1 except that two commercially available and non-prescription skin whitening products were assessed in a test where each was applied to opposite sides of a panelist's face. Forced-choice (self assessment) between photographs after twelve (12) weeks treatment and transforms were achieved with blue light transform and by blending 14% red channel into the blue channel. The D-prime values obtained for each product were about 0.4 after twelve (12) weeks of treatment, indicating that the products had similar skin lightening performances after twelve (12) weeks. Such D-prime values may be used to create a system with symbols to assist in the marketing and advertising of consumer products of non-prescription consumer products.

## Claims

1. A method for evaluating consumer products comprising the steps of:
(a) analyzing untreated skin of a consumer and artificially transforming the untreated skin to a desired result to produce artificially transformed skin by applying a cover-up or by using image transformation;
(b) treating the untreated skin with a product to produce treated skin; and
(c) comparing the treated skin to the artificially transformed skin to yield a difference associated with a symbol,
wherein at least two consumer products are evaluated and compared to artificially transformed skin,
wherein the method is not a method for performing a mental act, or for treatment of the human body by surgery ortherapy.

2. A method for evaluating consumer products according to clam 1, wherein the products are leave on orwash off products.

3. A method for evaluating consumer products according to claim 1 or claim 2, wherein the untreated, treated and artificially transformed skin are analyzed visually, by image analysis or through photographs.

4. A method for evaluating consumer products according to any one of the preceding claims, wherein the untreated skin is artificially transformed with digital transformation, or with transformation that uses light, lenses, a mirror, make-up or the morphing of a photograph.

5. A method for evaluating consumer products according to any one of the preceding claims, wherein the untreated skin is treated with a product that is suitable to be injected, ingested, topically applied, applied using radiation or applied with a physical device.

6. A method for evaluating consumer products according to any one of the preceding claims, wherein at least one of the products is a skin lightening product that comprises niacinamide.

7. A method for evaluating consumer products according to any one of the preceding claims, wherein the difference is statistically analyzed with D-prime analysis.

8. A method for evaluating consumer products according to any one of the preceding claims, wherein the products are evaluated for yielding evenness of skin color.

9. A method for evaluating consumer products according to claim 8, wherein the untreated skin is assessed for evenness of color and artificially transformed with blue light transform.

10. A method for evaluating consumer products according to claim 9, wherein from 0.5 to 60% red light is blended into blue light.

## Patentansprüche

1. Verfahren zum Bewerten von Konsumgütern, das die folgenden Schritte umfasst:
(a) Analysieren der unbehandelten Haut eines Verbrauchers und künstliches Transformieren der unbehandelten Haut in ein gewünschtes Ergebnis, um durch Aufbringen einer Abdeckung oder unter Verwendung einer Bildtransformation eine künstlich transformierte Haut zu erzeugen;
(b) Behandeln der unbehandelten Haut mit einem Produkt, um die behandelte Haut zu erzeugen; und
(c) Vergleichen der behandelten Haut mit der künstlich transformierten Haut, um einen Unterschied zu erhalten, der einem Symbol zugeordnet ist,
wobei mindestens zwei Konsumgüter bewertet und mit der künstlich transformierten Haut verglichen werden,
wobei das Verfahren kein Verfahren zum Durchführen eines mentalen Vorgangs oder zur Behandlung des menschlichen Körpers durch eine Operation oder durch eine Therapie ist.

2. Verfahren zum Bewerten von Konsumgütern nach Anspruch 1, wobei die Produkte permanente Produkte oder Produkte zum Abwaschen sind.

3. Verfahren zum Bewerten von Konsumgütern nach Anspruch 1 oder Anspruch 2, wobei die unbehandelte Haut, die behandelte Haut und die künstlich transformierte Haut optisch, durch eine Bildanalyse oder mit Hilfe von Fotografien analysiert werden.

4. Verfahren zum Bewerten von Konsumgütern nach einem der vorhergehenden Ansprüche, wobei die unbehandelte Haut mittels einer digitalen Transformation oder mittels einer Transformation, die Licht, Linsen, einen Spiegel, Make-Up oder das Umwandeln einer Fotografie verwendet, künstlich transformiert wird.

5. Verfahren zum Bewerten von Konsumgütern nach einem der vorhergehenden Ansprüche, wobei die unbehandelte Haut mit einem Produkt behandelt wird, das dafür geeignet ist, injiziert, eingenommen, oberflächlich aufgetragen, unter Verwendung von Strahlung aufgebracht oder mittels einer physischen Vorrichtung aufgebracht zu werden.

6. Verfahren zum Bewerten von Konsumgütern nach einem der vorhergehenden Ansprüche, wobei mindestens eines der Produkte ein Produkt zur Hautaufhellung ist, das Niacinamid umfasst.

7. Verfahren zum Bewerten von Konsumgütern nach einem der vorhergehenden Ansprüche, wobei der Unterschied mittels einer D-Prime-Analyse statistisch analysiert wird.

8. Verfahren zum Bewerten von Konsumgütern nach einem der vorhergehenden Ansprüche, wobei die Produkte nach dem Erzielen einer Gleichmäßigkeit der Hautfarbe bewertet werden.

9. Verfahren zum Bewerten von Konsumgütern nach Anspruch 8, wobei die unbehandelte Haut nach der Gleichmäßigkeit der Farbe bewertet und mittels einer Blaulichttransformation künstlich transformiert wird.

10. Verfahren zum Bewerten von Konsumgütern nach Anspruch 9, wobei 0,5 % bis 60 % rotes Licht in das blaue Licht gemischt wird.

## Revendications

1. Procédé d'évaluation de produits de consommation comprenant les étapes de :
(a) analyse de peau non traitée d'un consommateur et transformation artificielle de la peau non traitée jusqu'à un résultat souhaité pour produire une peau artificiellement transformée par application d'une couverture ou en utilisant une transformation d'image ;
(b) traitement de la peau non traitée avec un produit pour produire de la peau traitée ; et
(c) comparaison de la peau traitée à la peau artificiellement transformée pour produire une différence associée à un symbole,
dans lequel au moins deux produits de consommation sont évalués et comparés à une peau artificiellement transformée,
dans lequel le procédé n'est pas un procédé destiné à la réalisation d'un acte mental, ou au traitement du corps humain par chirurgie ou thérapie.

2. Procédé d'évaluation de produits de consommation selon la revendication 1, dans lequel les produits sont des produits à laisser ou sans rinçage.

3. Procédé d'évaluation de produits de consommation selon la revendication 1 ou la revendication 2, dans lequel les peaux non traitée, traitée et artificiellement transformée sont analysées visuellement, par analyse d'image ou par photographies.

4. Procédé d'évaluation de produits de consommation selon l'une quelconque des revendications précédentes, dans lequel la peau non traitée est artificiellement transformée avec une transformation digitale, ou avec une transformation qui utilise de la lumière, des lentilles, un miroir, du maquillage ou le morphing photographique.

5. Procédé d'évaluation de produits de consommation selon l'une quelconque des revendications précédentes, dans lequel la peau non traitée est traitée avec un produit qui est approprié pour être injecté, ingéré, topiquement appliqué, appliqué en utilisant un rayonnement ou appliqué avec un dispositif physique.

6. Procédé d'évaluation de produits de consommation selon l'une quelconque des revendications précédentes, dans lequel au moins un des produits est un produit éclaircissant la peau qui comprend du niacinamide.

7. Procédé d'évaluation de produits de consommation selon l'une quelconque des revendications précédentes, dans lequel la différence est statistiquement analysée avec une analyse D-prime.

8. Procédé d'évaluation de produits de consommation selon l'une quelconque des revendications précédentes, dans lequel les produits sont évalués pour produire une uniformité de couleur de peau.

9. Procédé d'évaluation de produits de consommation selon la revendication 8, dans lequel la peau non traitée est évaluée pour l'uniformité de couleur et est artificiellement transformée avec une transformation à la lumière bleue.

10. Procédé d'évaluation de produits de consommation selon la revendication 9, dans lequel de 0,5 à 60 % de lumière rouge sont mélangés dans de la lumière bleue.
